# FASCICULE DE BREVET EUROPEEN

(11) **EP 0 785 427 B1**
(45) Date de publication et mention de la délivrance du brevet: **15.10.2003**
(21) Numéro de dépôt: 96402900.3
(22) Date de dépôt: 26.12.1996
(51) Int. Cl.: G01N 33/18, G01N 27/00, G01N 27/42

(54) **Méthode et dispositif de contrôle en continu du pouvoir entartrant d'une eau**
Verfahren und Vorrichtung zur kontinuierlichen Überwachung des Ablagerungsvermögens von Wasser
Method and apparatus for continuously controlling the tendency to form a deposit of water

(30) Priorité: 17.01.1996 FR 9601253
(43) Date de publication de la demande: 23.07.1997
(73) Titulaire: Institut Français du Pétrole, 92852 Rueil-Malmaison Cedex (FR)
(72) Inventeur: Noik, Christine, 78230 Le Pecq (FR); Ropital, François, 92500 Rueil Malmaison (FR); Kouznetsov, Dmitri, Naperville, 60563 Illinois (US)

(56) Documents cités:
- EP-A- 0 504 730
- EP-A- 0 676 637

## Description

L'objet de ta présente invention se situe dans le domaine de l'analyse et du traitement des eaux industrielles, et plus précisément concerne un procédé et un dispositif pour évaluer le caractère entartrant d'eaux destinées à un recyclage.

La présente invention a donc pour but de permettre d'évaluer la potentialité de recyclage d'eaux provenant notamment de procédés chimiques ou pétrochimiques, dans le but de fournir des appoints d'eaux, par exemple, dans des circuits de réfrigération.

Il n'est pas actuellement facile d'effectuer des recyclages des eaux de raffineries au fur et à mesure de leur production, telles quelles ou après traitements adaptés (purification ou ajout d'additifs), vers des flux consommateurs d'eaux, car les méthodes d'évaluation en continu du pouvoir entartrant d'une eau ne sont pas adaptées.

On connaît le document EP-A-676637 qui décrit un dispositif pour la mesure de la variation de masse d'une électrode au cours d'une réaction électrochimique, le dispositif comprenant une microbalance à quartz. Ce document décrit un perfectionnement de l'appareil de mesure par une microbalance à quartz dont l'électrode de travail est balayée par un jet de forme spécifique.

Ainsi la présente invention concerne une méthode de contrôle du pouvoir entartrant d'une eau s'écoulant à travers un canal d'une géométrie déterminée, dans laquelle:
- on place dans ledit canal des moyens pour accélérer la précipitation de dépôts sur une électrode de travail, lesdits moyens comportant une électrode de référence) et au moins une électrode auxiliaire,
- on détermine les caractéristiques géométriques dudit canal en fonction de la nature de l'écoulement que l'on s'impose dans le voisinage de l'électrode de travail,
- on effectue, pendant un intervalle de temps fixé, une première mesure en fonction du temps de la masse des dépôts sur ladite électrode de travail à l'aide d'une microbalance à quartz dont l'une des électrodes est constituée par l'électrode de travail,
- on déduit de ladite mesure un paramètre lié au caractère entartrant dudit fluide dans les conditions de l'écoulement.

Selon la méthode:
- on peut effectuer une seconde mesure dans les mêmes conditions que la première,
- on peut interpréter la seconde mesure par rapport à la première pour déduire une évolution ou une stabilisation du pouvoir entartrant de ladite eau.
- on peut déduire de ladite mesure une vitesse de précipitation des dépôts.

Le canal peut avoir une section rectangulaire et une longueur suffisante pour que l'écoulement soit laminaire dans le voisinage de l'électrode de travail.

L'eau peut provenir d'un procédé de raffinage du pétrole situé en amont du canal.

L'eau peut être traitée en amont du canal dans une installation de traitement.

On peut piloter des moyens de distribution de l'eau situés en aval dudit canal, ladite eau étant distribuée soit vers un rejet, une utilisation ou un traitement supplémentaire en fonction des mesures.

L'invention concerne également un dispositif de contrôle du pouvoir entartrant d'une eau, comportant une microbalance à quartz, une électrode de référence, au moins une électrode auxiliaire, une électrode de travail constituée par l'une des électrodes de ladite microbalance. Dans le dispositif, l'électrode de travail est disposée dans un canal dans lequel circule l'eau, et le canal a des dimensions géométriques déterminées pour obtenir un type d'écoulement spécifique dans le voisinage de ladite électrode de travail.

Le canal peut comporter une portion de surface plane, l'électrode de travail étant placée sensiblement dans le même plan que ladite surface plane.

Le canal peut avoir une section rectangulaire.

La présente invention sera mieux comprise et ses avantages apparaîtront plus clairement à la description qui suit des exemples, nullement limitatifs, illustrés par les figures annexées, parmi lesquelles:
- la figure 1 montre une disposition de principe des différents moyens permettant un exemple de mise en oeuvre de la présente invention,
- la figure 2 montre le schéma de l'installation d'évaluation,
- la figure 3 montre le principe de l'électrode de travail disposée dans le canal selon l'invention.
- les figures 4A et 4B montrent un exemple des mesures effectuées par l'installation.

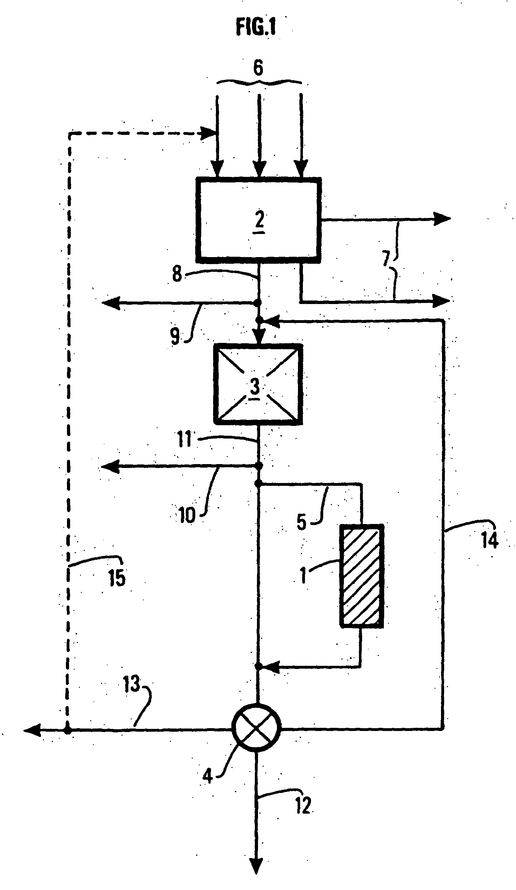

Sur la figure 1, les références 1, 2, 3 représentent respectivement le dispositif de contrôle du pouvoir entartrant selon l'invention, une installation de procédé de raffinage et un ensemble de moyens de traitement de l'eau. La référence 4 désigne des moyens de distribution du flux d'eau en provenance des moyens de traitement. Ces moyens de distribution sont situés en aval du flux d'eau qui provient de la dérivation 5 qui alimente le dispositif canal 1 selon la présente invention.

Les flèches 6 représentent les flux de matières en entrée du procédé, dont de l'eau. Les flèches 7 symbolisent les sorties des matériaux après procédé, la ligne référencée 8 étant la collecte d'eau qui peut être selon les cas, recyclée, rejetée, utilisée dans d'autres équipements, après ou sans traitement, en totalité ou en partie. Dans le présent exemple, l'eau en sortie par la collecte 8 est dirigée en totalité dans une installation de traitement de l'eau 3. Cette installation peut comporter tous les traitements classiques connus, y compris des adjonctions d'additifs.

Les références 9 et 10 représentent respectivement des conduites d'eau, avant et après traitement vers des utilisations diverses, par exemple d'autres procédés que le procédé 2, ou des installations nécessitant de l'eau comme des installations de réfrigération.

La conduite 11 de sortie de l'eau après traitement peut comporter une dérivation 5 qui conduit une partie de l'eau vers le dispositif selon l'invention 1.

En fonction des mesures apportées par le dispositif 1, les moyens de distribution 4 sont pilotés pour distribuer l'eau soit vers un rejet 12, soit une autre utilisation, un traitement d'eau secondaire 13 ou même un recyclage dans le procédé 2 par la ligne 15, soit vers un recyclage dans l'installation de traitement par la conduite 14.

Dans le cas où la capacité du dispositif 1 est telle qu'elle permet l'admission du débit total d'eau en sortie de l'installation de traitement 3, le dispositif pourra être placé directement en série sur la conduite 11.

Un tel schéma selon la figure 1 ne représente qu'un principe général d'utilisation du dispositif selon l'invention, d'autres variantes de distribution des flux et de leurs utilisations sont possibles dans le cadre de la présente invention.

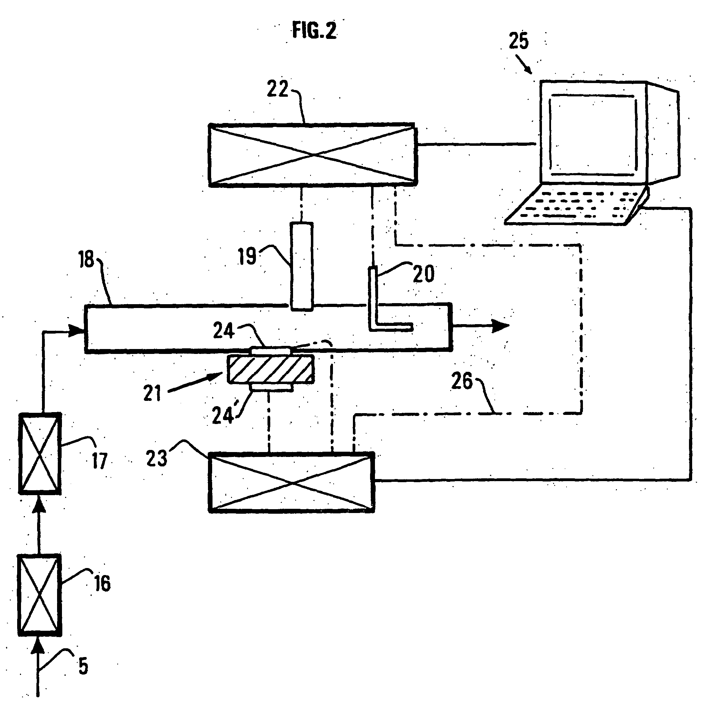

La figure 2 représente plus en détail les composants principaux du dispositif 1. Sur la dérivation 5, on dispose un débitmètre 16, des moyens de mesures 17 de la pression et de la température. Un canal 18, de forme spécifique, comporte une électrode de référence 19, au moins une électrode auxiliaire 20, toutes deux étant en liaison avec un potentiostat 22. Un cristal de quartz 21 constitue une microbalance dont l'une des électrodes 24 est en contact avec l'eau s'écoulant dans le canal. Cette électrode dite de travail est sensiblement placée en continuité de la paroi du canal, c'est à dire que la surface plane de l'électrode de travail est située dans le même plan que celui de la paroi intérieure du canal. Pour cela, dans le cas où la conduite de dérivation est cylindrique, on pourra disposer une surface plane dans la paroi intérieure. Les deux électrodes 24 et 24' sont reliées à un moniteur du quartz, permettant le contrôle de son oscillation et la mesure des dépôts sur l'électrode de travail 24. Une liaison électrique 26 entre le moniteur 23 et le potentiostat 22 permet de créer un potentiel électrochimique entre l'électrode de référence 19 et l'électrode de travail 24. La ou les électrodes auxiliaires 20 sont utilisées pour permettre la mesure de la variation d'intensité due aux dépôts sur l'électrode de travail. Un micro-ordinateur 25 pilote et traite les mesures obtenues par la microbalance et le potentiostat.

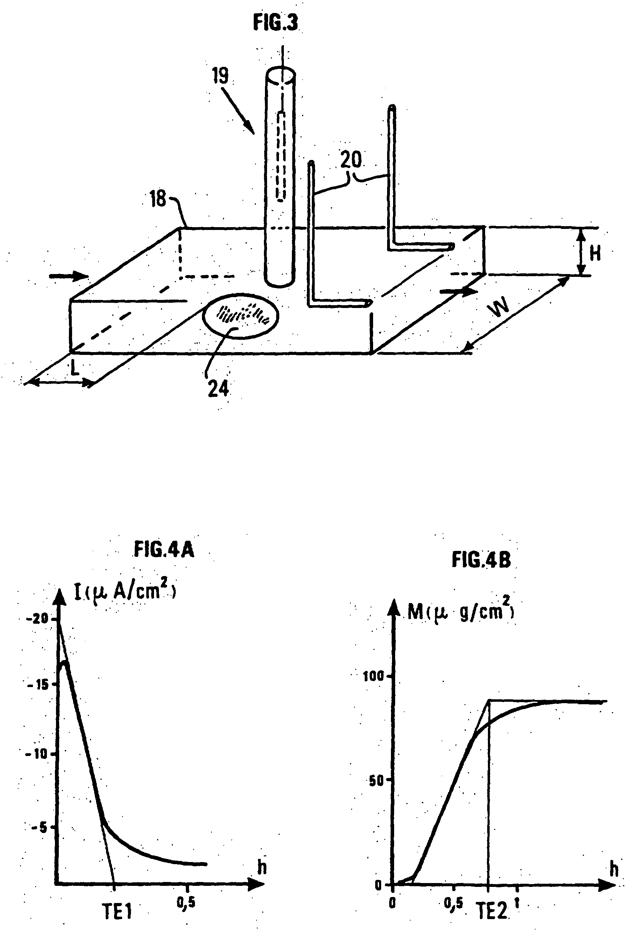

La figure 3 montre une réalisation préférentielle du canal 18. Celui-ci a une section rectangulaire de largeur W, de hauteur H et de longueur minimale L entre l'entrée du canal et l'électrode de travail 24.

La forme et la longueur du canal sont déterminées de façon à ce que l'écoulement de l'eau sur l'électrode de travail soit sensiblement laminaire et parallèle à la surface de l'électrode de travail. Ainsi, les mesures et les interprétations résultant des dépôts sur l'électrode 24 peuvent être représentatifs des conditions réelles dans lesquelles on pourra avoir des dépôts dans les installations industrielles.

Pour un débit de 1,5 litre/min, W=2,6 cm et H=0,15 cm, le nombre de Reynolds est environ 1570, la longueur minimale L étant 14,2 cm.

Pour un débit de 1,1 litre/min, W=2,6 cm et H=0,3 cm, le nombre de Reynolds est environ 1151, la longueur minimale L étant 20,7 cm.

L'électrode de travail est constituée par une couche d'or d'une surface circulaire d'environ 1,37 cm2. Le principe de la microbalance à quartz est basé sur la proportionnalité entre la variation de masse m déposée et la fréquence de résonance f d'une électrode en quartz. m=C.f avec C étant la constante de cellule égale à 1,77 10⁻⁸ g/sec.cm2.Hz pour une fréquence de résonance du quartz de 5 MHz. Le document EP-A-676637, cité en référence, décrit également le principe d'utilisation d'une microbalance à quartz.

Les figures 4A et 4 B montrent le principe des mesures que l'on peut effectuer avec le dispositif selon l'invention. La formation du tartre sur l'électcode de travail induit une impédance dans le circuit d'où une modification de l'intensité du courant, mesurée à l'aide de la combinaison des électrodes auxiliaires, de référence et de travail reliées au potentiostat 22. La figure 4A donne la forme générale de la courbe de la variation d'intensité (ordonnée) en fonction du temps (abscisse). L'analyse de la courbe intensité/temps donne accès à un temps d'entartrage TE1 défini comme indiqué sur la figure 4A. On définit un indice d'entartrage I=1000/TE1. Une eau peut être classée comme entartrante pour un indice supérieur à 15, et peu entartrante pour un indice compris entre 0,5 et 5. Une vitesse d'entartrage VE1 peut être calculée à partir de la pente de la courbe. Grâce au dispositif selon l'invention, on peut, en combinaison avec la réaction d'électrochimie, mesurer en continu la variation de masse de solides déposés sur l'électrode de travail. L'analyse de la courbe type représentée sur la figure 4B permet de distinguer trois zones:
- une première région I qui décrit la phase de germination qui correspond à l'apparition des premiers cristaux de carbonates. Un Eemps de germination TG est ainsi défini,
- une seconde région II reliée à une phase de croissance des germes et caractérisée par une vitesse d'entartrage VE2, correspondante à la pente de la courbe;
- un palier correspondant à une saturation de l'électrode par le carbonate après un temps TE2.

La variation de masse donne une indication sur la cinétique de formation du dépôt. Par exemple, suivant la structure chimique des additifs utilisés en traitement, certains peuvent agir sur la formation de germes de carbonate. Un temps de retard (temps de germination TG) par rapport à l'augmentation de masse est déterminé directement sur les courbes. De même, la croissance des cristaux peut être limitée par la présence ou non d'additifs. La vitesse d'entartrage VE peut également être modifiée.

Il est clair que l'invention permet d'analyser en continu ou sous forme de mesures prises ponctuellement pendant un laps de temps suffisant pour obtenir les informations nécessaires à la détermination du pouvoir entartrant ou non d'un flux d'eau, les mesures étant répétées à intervalle de temps régulier pour comparer les résultats des mesures ponctuelles. Les comparaisons et les interprétations des mesures permettent d'identifier également des évolutions du caractère entartrant du flux d'eau.

L'invention pourra être utilisée en cours de test d'un procédé de traitement d'hydrocarbure dans une unité pilote où un flux d'eau peut être collecté en sortie d'unité, la qualité de l'eau pouvant être une information technique nécessaire à l'industrialisation du procédé.

D'une manière industrielle, l'invention est avantageuse pour piloter en continu ou pseudo-continu, des procédés de raffinage, des traitements d'eau ou des installations de réfrigération.

## Revendications

1. Méthode de contrôle du pouvoir entartrant d'une eau s'écoulant à travers un canal (18) d'une géométrie déterminée, dans laquelle:
- on place dans ledit canal (18) des moyens pour accélérer la formation de tartre sur une électrode de travail (24), lesdits moyens comportant une électrode de référence (19) et au moins une électrode auxiliaire (20), la paroi dudit canal comportant une portion de surface plane, l'électrode de travail étant placée dans le même plan que ladite surface plane,
- on détermine les caractéristiques géométriques dudit canal en fonction de la nature de l'écoulement que l'on s'impose dans le voisinage de l'électrode de travail (24),
- on effectue, pendant un intervalle de temps fixé, une première mesure en fonction du temps de la masse du tartre sur ladite électrode de travail (24) à l'aide d'une microbalance à quartz (21) dont l'une des électrodes est constituée par l'électrode de travail,
- on déduit de ladite mesure un paramètre lié au caractère entartrant dudit fluide dans les conditions de l'écoulement.

2. Méthode selon la revendication 1, dans laquelle:
- on effectue une seconde mesure dans les mêmes conditions que la première,
- on interprète la seconde mesure par rapport à la première pour déduire une évolution ou une stabilisation du pouvoir entartrant de ladite eau.

3. Méthode selon l'une des revendications précédentes, dans laquelle on déduit de ladite mesure une vitesse de formation du tartre.

4. Méthode selon l'une des revendications précédentes, dans laquelle ledit canal a une section rectangulaire et une longueur suffisante pour que l'écoulement soit laminaire dans le voisinage de l'électrode de travail (24).

5. Méthode selon l'une des revendications précédentes, dans laquelle ladite eau provient d'un procédé (2) de raffinage du pétrole situé en amont dudit canal (18).

6. Méthode selon l'une des revendications précédentes, dans laquelle ladite eau est traitée en amont dudit canal dans une installation de traitement (3).

7. Méthode selon l'une des revendications précédentes, dans laquelle on pilote des moyens de distribution (4) de l'eau situés en aval dudit canal, ladite eau étant distribuée soit vers un rejet (12), une utilisation ou un traitement supplémentaire en fonction desdites mesures.

8. Dispositif de contrôle du pouvoir entartrant d'une eau, comportant une microbalance à quartz (21), une électrode de référence (19), au moins une électrode auxiliaire (20), une électrode de travail (24) constituée par l'une des électrodes de ladite microbalance, **caractérisé en ce que** ladite électrode de travail est disposé dans un canal (18) dans lequel circule ladite eau, **en ce que** ledit canal a des dimensions géométriques déterminées pour obtenir un type d'écoulement spécifique dans le voisinage de ladite électrode de travail, et **en ce que** la paroi dudit canal comporte une portion de surface plane, l'électrode de travail étant placée dans le même plan que ladite surface plane.

9. Dispositif selon la revendication 8, dans lequel le canal a une section rectangulaire.

## Patentansprüche

1. Verfahren zum Regeln des Kesselsteinansatzverhaltens eines durch einen Kanal (18) bestimmter Geometrie strömenden Wassers, bei dem:
a. man in diesen Kanal (18) Mittel einsetzt, um die Bildung von Kesselstein an einer Arbeitselektrode (24) zu beschleunigen, wobei diese Mittel eine Bezugselektrode (19) und wenigstens eine Hilfselektrode (20) umfassen, die Wandung dieses Kanals einen Teil mit planer Oberfläche umfasst und die Arbeitselektrode in der gleichen Ebene wie diese plane Fläche angeordnet ist,
b. man die geometrischen Charakteristiken dieses Kanals als Funktion der Natur der Strömung bestimmt, die man benachbart der Arbeitselektrode (24) aufbringt,
c. man während eines festgelegten Zeitintervalls eine erste Messung als Funktion der Zeit der Kesselsteinmasse an dieser Arbeitselektrode (24) mit Hilfe einer Quarzmikrowaage (21), deren eine Elektrode durch die Arbeitselektrode gebildet wird, vornimmt und
d. man aus dieser Messung einen Parameter herleitet, der mit dem Kesselsteinansatzvermögen dieses Fluids unter Strömungsbedingungen verknüpft ist.

2. Verfahren nach Anspruch 1, bei dem:
a. man eine zweite Messung unter den gleichen Bedingungen wie die erste vornimmt und
b. man die zweite Messung, bezogen auf die erste, interpretiert, um eine Entwicklung oder Stabilisierung des Kesselsteinansatzvermögens des Wassers herzuleiten.

3. Verfahren nach einem der vorhergehenden Ansprüche, bei dem man aus dieser Messung eine Kesselsteinbildungsgeschwindigkeit herleitet.

4. Verfahren nach einem der vorhergehenden Ansprüche, bei dem dieser Kanal über einen Rechteckquerschnitt und eine ausreichende Länge verfügt, damit die Strömung laminar benachbart der Arbeitselektrode (24) wird.

5. Verfahren nach einem der vorhergehenden Ansprüche, bei dem dieses Wasser aus einem Verfahren der anströmseitig zu diesem Kanal (18) befindlichen Erdölraffinierung stammt.

6. Verfahren nach einem der vorhergehenden Ansprüche, bei dem dieses Wasser vor diesem Kanal in einer Verarbeitungsanlage (3) behandelt wird.

7. Verfahren nach einem der vorhergehenden Ansprüche, bei dem man Verteilermittel (4) für das Wasser, die hinter diesem Kanal angeordnet sind, (hilfs)steuert, wobei das Wasser entweder gegen einen Austrag (12), eine Verwendung oder eine zusätzliche Behandlung als Funktion dieser Messungen verteilt wird.

8. Verfahren zur Regelung des Kesselsteinansatzvermögens eines Wassers, umfassend: eine Quarzmikrowaage (21), eine Bezugselektrode (19), wenigstens eine Hilfselektrode (20) und eine Arbeitselektrode (24), die durch eine der Elektroden der Mikrowaage bestimmt wird, **dadurch gekennzeichnet, dass** diese Arbeitselektrode in einem Kanal (18), in welchem dieses Wasser zirkuliert, angeordnet ist, dass dieser Kanal Abmessungen von einer bestimmten Geometrie zum Erhalt eines spezifischen Strömungstyps benachbart dieser Arbeitselektrode hat und dass die Wandung dieses Kanals einen planen Oberflächenteil umfasst, wobei die Arbeitselektrode in der gleichen Ebene wie diese plane Fläche angeordnet ist.

9. Vorrichtung nach Anspruch 8, bei der der Kanal einen Rechteckquerschnitt hat.

## Claims

1. A method for monitoring the scaling power of a water flowing through a channel (18) of determined geometry, wherein :
- means for accelerating scale formation on a working electrode (24) are placed in said channel (18), said means comprising a reference electrode (19) and at least one auxiliary electrode (20), the wall of said channel comprising a plane surface portion, the working electrode being arranged in the same plane as said plane surface,
- the geometric characteristics of said channel are determined according to the nature of the flow applied in the vicinity of working electrode (24),
- during a fixed time interval, a first measurement is made, as a function of time, of the mass of scale on said working electrode (24) by means of a quartz microbalance (21), one of whose electrodes consists of the working electrode,
- a parameter related to the scaling property of said fluid under the conditions of flow is deduced from said measurement.

2. A method as claimed in claim 1, wherein:
- a second measurement is performed under the same conditions as the first one,
- the second measurement is interpreted in relation to the first one to deduce an evolution or a stabilization of the scaling power of said water.

3. A method as claimed in any one of the previous claims, wherein a scale formation rate is deduced from said measurement.

4. A method as claimed in any one of the previous claims, wherein said channel has a rectangular section and a sufficient length for the flow to be laminar in the vicinity of working electrode (24).

5. A method as claimed in any one of the previous claims, wherein said water comes from an oil refining process (2) upstream from said channel (18).

6. A method as claimed in any one of the previous claims, wherein said water is treated upstream from said channel in a treating facility (3).

7. A method as claimed in any one of the previous claims, wherein water distribution means (4) arranged downstream from said channel are controlled, and said water can be sent either to a discharge (12), to use or to additional treatment according to said measurements.

8. A device for monitoring the scaling power of a water, comprising a quartz microbalance (21), a reference electrode (19), at least one auxiliary electrode (20), a working electrode (24) consisting of one of the electrodes of said microbalance, **characterized in that** said working electrode is arranged in a channel (18) in which said water circulates, **in that** said channel has geometric dimensions selected to obtain a specific flow type in the vicinity of said working electrode, and **in that** the wall of said channel comprises a plane surface portion, the working electrode being arranged in the same plane as said plane surface.

9. A device as claimed in claim 8, wherein the channel has a rectangular section.
